# EUROPÄISCHE PATENTSCHRIFT

(11) **EP 0 334 283 B1**
(45) Veröffentlichungstag und Bekanntmachung des Hinweises auf die Patenterteilung: **22.09.1993**
(21) Anmeldenummer: 89105013.0
(22) Anmeldetag: 21.03.1989
(51) Int. Cl.: C12N 15/10

(54) **Verfahren zur Selektion grosser DNA-Abschnitte**
Process for the selection of large DNA segments
Procédé de sélection de larges segments d'ADN

(30) Priorität: 23.03.1988 DE 3809691
(43) Veröffentlichungstag der Anmeldung: 27.09.1989
(73) Patentinhaber: HOECHST AKTIENGESELLSCHAFT, 65926 Frankfurt am Main (DE)
(72) Erfinder: Wohlleben, Wolfgang, Dr., D-4800 Bielefeld (DE); Muth, Günter, Dr., D-4800 Bielefeld (DE); Pühler, Alfred, Prof. Dr., D-4800 Bielefeld (DE)

(56) Entgegenhaltungen:
- EP-A- 0 243 856
- EP-A- 0 334 282
- E.L. WINNACKER: "From Genes to Clones", 1987, Seiten 383-387, VCH Verlagsgesellschaft, Weinheim, DE

## Beschreibung

Aus der europäischen Patentanmeldung mit der Veröffentlichungsnummer (EP-A) 0 243 856 ist ein Verfahren zur Herstellung von Mutanten bekannt, bei dem man aus dem Ausgangsstamm die gesamte DNA isoliert, sie in kurze Fragmente überführt, diese in ein Plasmid integriert, das einen Marker enthält, temperatursensitiv ist und in dem Ausgangsstamm repliziert, die erhaltene Hybridplasmid-Population in den Ausgangsstamm transformiert, durch Selektion auf den Marker die Transformanten selektiert, durch Erhöhung der Temperatur über den Schwellenwert des temperatursensitiven Plasmids die Hybridplasmide eliminiert und durch erneute Selektion auf den Marker die Mutanten selektiert. Bei diesem letzten Selektionsschritt werden also nur Zellen erhalten, die Plasmid-DNA mit dem Marker in das Chromosom aufgenommen haben. Da die Integration der Plasmid-DNA bevorzugt über die in das Plasmid klonierte DNA in eine entsprechende homologe DNA-Region der Wirtszelle erfolgt, führt dieser Vorgang dann zu einer Inaktivierung eines Gens in dieser homologen Region, wenn das in dem Plasmid klonierte DNA-Fragment weder die Promotorregion noch die Signale für den Translationsstop dieses Gens enthält. Wird nun das Genom der so erhaltenen Mutante in eine Genbank überführt, so gewinnt man durch Selektion auf den Marker direkt die Klone, die das mutierte Gen enthalten.

Im Gegensatz zu dem schon länger bekannten Verfahren unter Verwendung des Phagen ØC31, bei dem nur kurze DNA-Fragmente isoliert werden können, erlaubt das Verfahren nach der EP-A 0 243 856 die Isolierung etwas längerer DNA-Abschnitte, die beispielsweise neben dem mutierten Gen benachbarte unmutierte Gene umfassen können. Die Voraussetzung hierfür ist allerdings, daß geeignete Schnittstellen für Restriktionsenzyme zur Verfügung stehen und daß die gewünschten Gene auf dem klonierten DNA-Abschnitt in der erhofften Weise angeordnet sind.

Es wurde nun gefunden, daß die geschilderten Beschränkungen bezüglich der Länge und der Anordnung von Restriktionsschnittstellen des zu isolierenden mutierten DNA-Fragments und seiner benachbarten Regionen überwunden werden können, wenn man bei diesem Verfahren einen Marker wählt, der in E. coli selektionierbar ist. Man kann dann nämlich das mutierte Gen direkt aus einer Cosmid-Genbank in E. coli selektionieren. Dazu legt man eine Cosmid-Genbank der Mutante in E. coli an. Nach Selektion auf den integrierten Marker, zweckmäßig gleichzeitig mit der Selektion auf den Cosmid-eigenen Marker, wird unmittelbar das interessierende Cosmid selektiert. Das sonst erforderliche, sehr aufwendige "Screening" durch Hybridisierung wird dadurch überflüssig.

Der Begriff "Cosmid" soll hierbei neben den eigentlichen Cosmiden gleichwirkende E. coli-Plasmide umfassen, die - wie Cosmide - in der Lage sind, DNA-Fragmente von etwa 40-50 kb stabil zu integrieren und zu replizieren. Besonders geeignet sind solche Plasmide, die in geringer Kopienzahl in der Zelle vorliegen.

Das erfindungsgemäße Verfahren gestattet somit die Isolierung großer DNA-Regionen (in der Größenordnung von 40 kb), wie sie in an sich bekannter Weise in Cosmide integrierbar sind. Dieses Verfahren eignet sich damit besonders für die Isolierung von Gen-"Clustern" und erleichtert die Aufklärung von ganzen Biosynthesewegen, deren Gene häufig in der Form von "Clustern" angeordnet sind.

Der in E. coli selektionierbare Marker muß nicht mit dem Marker übereinstimmen, der für die ersten Selektionsschritte des bekannten Verfahrens herangezogen wird. Man kann vielmehr für das erfindungsgemäße Verfahren temperatursensitive oder temperatursensitiv gemachte Plasmide einsetzen, die einerseits über Marker verfügen, die in E. coli selektionierbar sind, und andererseits über DNA-Bereiche verfügen, die in dem zu mutierenden Stamm replizieren und selektiert werden können. Die Konstruktion solcher Vektoren ist bekannt.

Resistenzmarker, die sowohl in E. coli als auch beispielsweise in Streptomyceten vom eigenen Promotor exprimiert werden, sind z. B. die Gene für die Kanamycin-und die Gentamicin-Resistenz (EP-A 0 248 207).

Bezüglich der weiteren Einzelheiten kann auf die EP-A 0 243 856 verwiesen werden.

Eine bevorzugte Ausführungsform der Erfindung betrifft die Herstellung und Isolierung von Streptomyceten-Mutanten. Hierfür kann besonders vorteilhaft das Plasmid pSG5 eingesetzt werden, das aus der europäischen Patentschrift (EP-B) 0 158 872 (bzw. der veröffentlichten australischen Anmeldung 40599/85) bekannt ist. Es zeigte sich nämlich überraschenderweise, daß dieses Plasmid temperatursensitiv ist und deshalb nach Einfügung eines Markers unmittelbar für das erfindungsgemäße Verfahren herangezogen werden kann. Auf die Verwendung des Plasmids pSG5 als temperatursensitives Plasmid bezieht sich die am gleichen Tage eingereichte Patentanmeldung mit der Bezeichnung "Verwendung von pSG5 als temperatursensitives Plasmid" (HOE 88/F 069, entsprechend der deutschen Patentanmeldung P 38 09 692.7 vom 23.3.1988).

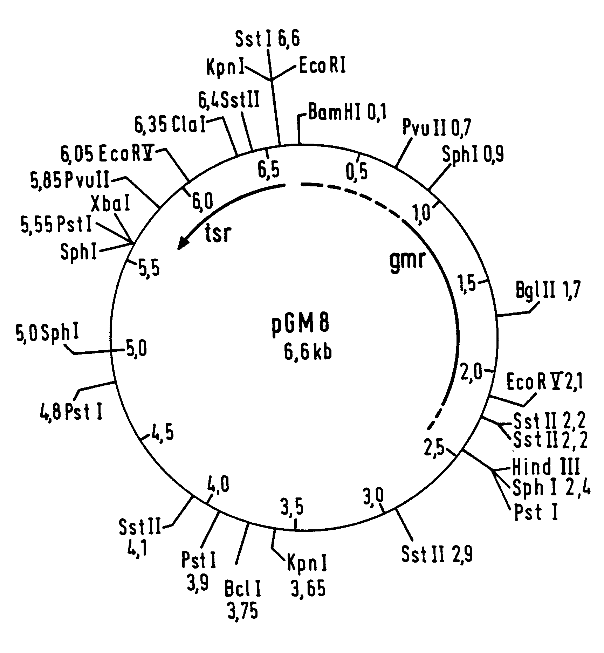

Die Figur zeigt das Plasmid pGM8, ein Derivat von pSG5 mit zwei Marker.

In den folgenden Beispielen wird die Erfindung näher erläutert.

### Beispiel 1

### Konstruktion des Streptomycetenvektors pGM8

Der temperatursensitive Vektor pGM8 ist ein Derivat des Plasmids pSG5, das aus der EP-B 0 158 872 bekannt ist (und unter den Bedingungen des Budapester Vertrags bei der Deutschen Sammlung von Mikroorganismen und Zellkulturen unter Nr. DSM 2932 hinterlegt ist). In dieser EP-B sind bereits zwei Vektoren beschrieben, die sich vom pSG5 ableiten und Antibiotika-Resistenzgene tragen. In analoger Weise erfolgt die Konstruktion von pGM8:

Zunächst isoliert man aus dem handelsüblichen Plasmid pIJ6 - wie in der EP-B 0 158 872 beschrieben - mit BclI ein 1,1 kb Fragment, auf dem das Thiostrepton-Resistenzgen tsr liegt. Dieses Fragment wird in den handelsüblichen Vektor pUC19 ligiert, der mit BamHI geöffnet wurde. Man erhält so das Plasmid pSLE60.

pSLE60 wird zunächst mit HindIII geöffnet, worauf die überstehenden Enden mit Klenow-Polymerase aufgefüllt werden. Hierauf wird mit EcoRI geschnitten und das 1,1 kb Fragment isoliert, auf dem das tsr-Gen liegt.

Das Plasmid pSG5 wird nun mit EcoRI und PvuII total verdaut und das 3,2 kb Fragment isoliert, das den Replikationsursprung trägt. Dieses Fragment wird nunmehr mit dem Fragment ligiert, daß das tsr-Gen trägt. Man erhält so das 4,3 kb große Plasmid pGM5.

In pGM5 wird nunmehr als zweiter Resistenzmarker ein Gentamicin-Resistenzgen eingesetzt, das in Streptomyceten und in E. coli aktiv ist. Hierzu wird aus dem Plasmid pSLE80 (vgl. EP-A 0 248 207; hinterlegt unter den Bedingungen des Budapester Vertrags unter Nr. DSM 3710) mit SalI das 2,3 kb Fragment isoliert, daß das Gm^{r}-Gen trägt. pGM5 wird nun mit XhoI geöffnet und mit dem 2,3 kb Fragment ligiert, wobei der Vektor pGM8 erhalten wird. In diesem stehen Schnittstellen für EcoRI, SstI, HindIII, XbaI, ClaI (Inaktivierung von tsr) und BglII (Inaktivierung von Gm^{r}) zur Verfügung.

Die Figur zeigt die Restriktionskarte von pGM8.

### Beispiel 2

Als Ausgangsmaterial dient der Stamm S. hygroscopicus ATCC 21705, der in den EP-A 0 173 372, 0 242 236 und 0 242 246 genannt und allgemein zugänglich ist. Dieser Stamm enthält ein Resistenzgen gegen das Antibiotikum Phosphinothricyl-alanyl-alanin (PTT). Aus Thompson et al., EMBO J. 6 (9), 2519-2523, 1987, ist bekannt, daß durch eine Doppelverdauung mit SalI und KpnI ein DNA-Fragment entsteht, das Teile des Resistenzgens ohne Promotor und ohne Translationsstop umfaßt.

Hierzu isoliert man die Gesamt-DNA aus dem genannten Stamm, spaltet die DNA mit SalI und KpnI und isoliert ein DNA-Fragment der Größe von etwa 250 bp. Diese DNA wird in dem handelsüblichen Vektor pUC18 amplifiziert, der mit SalI und KpnI geöffnet wurde. Nach der Amplifizierung wird das DNA-Fragment mit dem Resistenzgen mit HindIII und EcoRI ausgeschnitten und - nach Anbringen eines entsprechenden Linkers - entweder in die EcoRI- oder in die HindIII-Schnittstelle des Streptomycetenvektors pGM8 eingesetzt.

Das Ligationsgemisch wird nach S. hygroscopicus transformiert. Plasmidtragende Zellen selektioniert man auf Thiostrepton-und/oder Gentamicin-Resistenz. Die Transformanten werden auf nicht-selektivem Medium bei einer Temperatur von oberhalb 36°C inkubiert, um alle autonomen Plasmide zu eliminieren. Die erneute Selektion gestempelter Sporen auf Selektionsmedium liefert nur solche Kolonien, die Plasmid-DNA in das Chromosom integriert haben. Aufgrund der bevorzugten Rekombination über die klonierten homologen Bereiche handelt es sich bei den thiostreptonresistenten Zellen im wesentlichen um Mutanten, aus denen nun die PTT-sensitiven Zellen selektioniert werden können.

### Beispiel 3

### Isolierung des mutierten PTT-Resistenzgens und benachbarter DNA-Regionen

Von den nach Beispiel 2 erhaltenen Mutanten legt man mit Hilfe des handelsüblichen Cosmids pHC79 (Boehringer Mannheim; vgl. E.-L. Winnacker, Gene und Klone, VCH Verlagsgesellschaft, Weinheim, 1985, S. 150) eine Genbank an. Durch gleichzeitige Selektion auf Gentamicin- und Tetracyclin- oder Ampicillinresistenz erhält man direkt die Klone, die das mutierte Gen und die umliegenden DNA-Regionen tragen. Da aus Murakami et al., Mol. Gen. Genet. 205, 42-50, 1986, bekannt ist, daß das PTT-Resistenzgen als Teil des 16 kb Biosynthese-"Clusters" vorliegt, erhält man so auf direktem Weg Cosmide, die diese Biosynthesegene tragen.

## Patentansprüche

1. Verfahren zur Selektion von DNA-Abschnitten aus Mutanten, wobei man aus einem zu mutierenden Ausgangsstamm die gesamte DNA isoliert, in kurze Fragmente überführt, diese in ein Plasmid integriert, welches temperatursensitiv ist, einen Selektionsmarker enthält und in dem Ausgangsstamm repliziert, die so erhaltene Hybridplasmid-Population in den Ausgangsstamm transformiert, durch Selektion auf den Marker die Transformanten selektiert, durch Erhöhen der Temperatur über den Schwellenwert des temperatursensitiven Plasmids die Hybridplasmide eliminiert, durch erneute Selektion auf den Marker die Mutanten mit in das Genom integrierter Plasmid-DNA isoliert, aus der DNA der Mutanten eine Genbank anlegt und durch Selektion auf den Marker Klone mit dem mutierten Gen selektiert, dadurch gekennzeichnet, daß das temperatursensitive Plasmid einen in E. coli exprimierenden Selektionsmarker enthält und daß die mit der DNA der Mutanten angelegte Genbank eine Cosmid-Bank ist.

2. Verfahren nach Anspruch 1, dadurch gekennzeichnet, daß der in E. coli exprimierbare Selektionsmarker identisch ist mit dem, der für die Selektion der Transformanten im Ausgangsstamm herangezogen wird.

3. Verwendung des Verfahrens nach Anspruch 1 oder 2 zur Isolierung von Gen-"Clustern".

## Claims

1. A method for the selection of DNA sections from mutants, entailing the complete DNA being isolated from an initial strain which is to be mutated and being converted into short fragments, the latter being integrated into a plasmid which is temperature-sensitive, contains a selection marker and replicates in the initial strain, the hybrid plasmid population obtained in this way being transformed into the initial strain, the transformants being selected by selection for the marker, the hybrid plasmids being eliminated by increasing the temperature above the threshold of the temperature-sensitive plasmid, the mutants having the plasmid DNA integrated into the genome being isolated by renewed selection for the marker, a gene bank being constructed from the DNA of the mutants, and clones having the mutated gene being selected by selection for the marker, wherein the temperature-sensitive plasmid contains a selection marker expressing in E. coli, and wherein the gene bank constructed with the DNA of the mutants is a cosmid bank.

2. The method as claimed in claim 1, wherein the selection marker expressible in E. coli is identical to that used for the selection of the transformants in the initial strain.

3. The use of the method as claimed in claim 1 or 2 for isolating gene clusters.

## Revendications

1. Procédé pour la sélection de segments d'ADN à partir de mutants, dans lequel on isole l'ADN complet à partir d'une souche initiale à muter, on le convertit en courts fragments, on les intègre dans un plasmide qui est sensible à la température, contient un marqueur de sélection et se réplique dans la souche initiale, la population de plasmides hybrides ainsi obtenue est transformée dans la souche initiale, on sélectionne les transformants par sélection sur la base du marqueur, on élimine les plasmides hybrides par élévation de la température au-dessus du seuil du plasmide sensible à la température, on isole, par nouvelle sélection sur la base du marqueur, les mutants comportant de l'ADN plasmidique intégré dans le génome, on construit une banque de gènes à partir de l'ADN des mutants et, par sélection sur la base du marqueur, on sélectionne des clones comportant le gène muté, caractérisé en ce que le plasmide sensible à la température contient un marqueur de sélection s'exprimant dans *E. coli* et en ce que la banque de gènes construite avec l'ADN des mutants est une banque de cosmides.

2. Procédé selon la revendication 1, caractérisé en ce que le marqueur de sélection exprimable dans *E. coli* est identique à celui auquel on fait appel pour la sélection des transformants dans la souche initiale.

3. Utilisation du procédé selon la revendication 1 ou 2, pour l'isolement de "groupes" de gènes.
